# EUROPEAN PATENT APPLICATION

(11) **EP 3 796 022 A1**
(43) Date of publication of application: **24.03.2021**
(21) Application number: 19198528.2
(22) Date of filing: 20.09.2019
(51) Int. Cl.: G01R 33/38, G01R 33/3815, G16H 40/40

(54) **COLD-HEAD MONITORING**

(71) Applicant: Koninklijke Philips N.V., 5656 AG Eindhoven (NL)
(72) Inventor: PUTKINEN, Eero, 5656 AE Eindhoven (NL)
(74) Representative: de Haan, Poul Erik

(57) **Abstract**

A cold-head monitoring system (400) for monitoring operation of a cold-head (30) of a superconducting magnet cooling device (300), comprises a sound detection device (4) positioned such that it is able to detect sound generated by the cold-head (30). It further comprises a sound analyzer (41) that is configured to analyze a sound characteristic of the detected sound from the cold-head (30). It further comprises a transmitter (42) arranged to transmit the determined analyzed sound characteristics and/or a report thereof to a maintenance or servicing engineer.

## Description

### FIELD OF THE INVENTION

The present invention generally relates to a cold-head monitoring system for monitoring operation of a cold-head of a superconducting magnet cooling device, a cooling system for cooling a superconducting magnet, a method for monitoring operation of a cold-head of a superconducting magnet cooling device and a cold-head servicing workflow organizer.

### BACKGROUND OF THE INVENTION

In (medical) magnetic resonance imaging (MRI) strong magnetic fields are generated. When a body is placed in such a magnetic field atomic nuclei inside the body and their associated magnetic vector are oriented. The magnetic field is changed by introducing radio waves to the magnetic field causing the protons to resonate. Antennas placed in proximity to the body detect the resulting radio-frequency signal, thereby localizing the nuclei. A three-dimensional image based on atomic nuclei locations is formed. Hydrogen nuclei in for instance fat or water may be distinguished by using different modulations of the magnetic filed (sequences).

To be able to orient the nuclei properly magnetic fields from 0,5T or higher are used (up to and over 7T magnets are available). Such magnetic fields are generated by superconducting magnets that need to be cooled to a few degrees above the absolute minimum. To achieve this a cooling system comprising thousands of liters of liquid helium is installed. As the helium absorbs energy from the magnet, its temperature will increase above boiling point (4.2 K) and the helium will evaporate to its gaseous state. This is undesired as this reduces the efficiency of the cooling system and the gaseous helium needs to be vented when a too high pressure is obtained. To avoid, or at least reduce, this effect a so-called cold-head is installed within the cooling system. The cold-head re-condenses formed helium gas back to the liquid state and prevents efficiency and helium loss.

During use cold-head performance drops over time and when the cooling capacity becomes too low the cold-head is not capable anymore of re-condensing the gaseous helium and the performance of the MRI will deteriorate and/or helium needs to be vented. To prevent this the cold-head needs to be regularly serviced in person by a service engineer that travels to the site of the MRI and checks the cold-head and, if necessary, services or replaces it. This is time consuming and expensive. It would be beneficial if a service engineer would only need to visit a site when it is known that a cold-head needs servicing or replacing. While it is possible to have standard service moment or replacement after a pre-determined amount of operating time, this is also not optimal as not all cold-heads deteriorate at the same rate and servicing or replacement would have been overdue or not necessary at the time the service engineer is present.

### SUMMARY OF THE INVENTION

Embodiments according to the present invention are directed to a cold-head monitoring system for monitoring operation of a cold-head of a superconducting magnet cooling device. The system comprises a sound detection device positioned such that it is able to detect sound generated by the cold-head. The system further comprises a sound analyzer that is configured to analyze a sound characteristic of the detected sound from the cold-head. The system further comprises a transmitter arranged to transmit the determined analyzed sound characteristics and/or a report thereof to a maintenance or servicing engineer. Sound characteristics of sound generated by a cold-head may indicate sub-optimal or failing cold-head operation. As such by detecting and analyzing the sound a service or maintenance engineer may remotely monitor cold-head operation and may be warned timely about any potential operational problem and may therefore schedule to visit only when servicing is necessary. A further advantage of using such a monitoring system is that the sound detection device may be a separate device not directly connected to the imaging system or supporting equipment. It remotely monitors, even when closely located, operation of the cold-head and therefore is not part of the medical system itself and is therefore medical regulation is more lenient or not even needed.

In an embodiment the sound analyzer is configured to analyze the sound characteristics at pre-determined regular intervals and/or when requested by maintenance or servicing engineer. As such a regular check of the cold-head is performed thereby ensuring early detection of potential issues. An engineer may also decide to request a sound analysis on demand, for instance if he thinks there may be a problem, e.g. because a cold-head with similar specifications failed elsewhere recently.

In an embodiment the sound analyzer is configured to determine if the sound characteristic is within a pre-determined range of a predetermined reference sound characteristic of the cold-head that is associated with normal operation. A determination of sound characteristics of the cold-head operating at optimal conditions, e.g. when it was newly installed or a known sound characteristic profile valid for multiple cold-heads, provides a baseline for optimal operation. During use cold-heads operation will deteriorate due to aging or damage. When one or more sound characteristics deviate beyond the threshold it is an indication of sub-optimal performance for which servicing is required. The threshold prevents that the service engineer is notified for only minor fluctuations or for a decrease in performance that still is acceptable for operating a cooling system.

In an embodiment the transmitter transmits a notable alert when the sound characteristic is outside the pre-determined range. A clear alert ensures that the engineer is aware that he is aware that a cold-head may need servicing. If this is not clear then the engineer may accidentally miss the notification that a cold-head is not operating optimally.

In an embodiment the sound characteristic includes any of the following: a frequency, a volume, a silence, an additional single, continuous or intermittent sound that is not present during normal operation of the cold-head. These are sound characteristics that all may relate to proper operation and are all easily determined once a sound is detected and transmitted to the sound analyzer.

In an embodiment the sound analyzer is configured to analyze the detected sound during a time interval with a predetermined length, preferably a length less than 10 minutes, more preferably between 5 and 10 minutes. By analyzing an extended period of time chances are greater that irregular sound characteristics are detected or that a change of sound characteristics is observed.

In an embodiment the sound detection device is arranged to detect sound from the cold-head with as little as possible ambient sounds not generated by the cold-head, for instance by using a directional microphone aimed at the sound-head and/or shielding provided to shield the ambient sounds from reaching the sound detection device. In another embodiment the sound analyzer is configured to separate ambient sounds not generated by the cold-head from sound generated by the cold-head and then preferably disregard the ambient sounds in the analysis of the detected sound and/or include information of the ambient sounds in the information to be transmitted by the transmitter. As such it is ensured that no false warnings caused by sounds not generated by the cold-head are notified to the engineer.

Another embodiment of the present invention is directed towards a cooling system for cooling a superconducting magnet comprising a cooling device comprising a cold-head (30); and a cold-head monitoring system for monitoring operation of the cold-head as described for the embodiments above.

In an embodiment the cooling device further comprises a compressor for compressing helium and to supply pressurized, preferably liquid, helium to the cold-head through a high pressure helium conduit and to receive depressurized helium from the cold-head through a low pressure helium conduit. This is a commonly used cry-system for supercooling magnets, for instance in an MR imager, which is also an embodiment of the presently claimed invention.

A further embodiment of the present invention is directed towards a method for monitoring operation of a cold-head of a superconducting magnet cooling device, comprising: detecting a sound generated by the cold-head; analyze a sound characteristic of the detected sound from the cold-head; and transmit the determined analyzed sound characteristics and/or a report thereof to a maintenance or servicing engineer.

Another embodiment of the present invention is directed towards a cold-head servicing workflow organizer comprising: a receiver for receiving operational information from a cold-head of a cooling system for cooling for a superconducting magnet, wherein the operational information is supplied by a cold-head monitoring system as claimed by the present invention; a determination unit for determining based on the received operational information whether servicing is necessary; and a notification unit for providing (202) a report about the operational information and in case servicing was deemed necessary, notifying a service engineer that servicing of the cold-head is required. Such a workflow organizer may assist in providing an optimal servicing schedule for the service engineer.

Preferably the receiver is arranged to receive operational information from at least two cold-heads of different cooling systems. Preferably the organizer further comprises a scheduling unit arranged to schedule one or more visits to service one or multiple cold-heads based on geographical location of the cold-head or cold-heads and urgency of necessary servicing. As such the servicing schedule may be optimized with respect to multiple cold-heads and/or with at little as unnecessary travel as possible and urgent cases may be serviced sooner.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

### BRIEF DESCRIPTION OF THE DRAWINGS

The present invention is illustrated by drawings of which
Fig. 1 shows a schematic overview of an MRI with its cryogenic cooling unit, including a cold-head.
Fig. 2 shows a schematic overview of a cold-head and a remote cold-head monitoring system according to the present invention.
Fig. 3 shows a flow chart of a method to monitor a cold-head according to the present invention.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may be not be according to scale.

### DETAILED DESRIPTION OF EMBODIMENTS

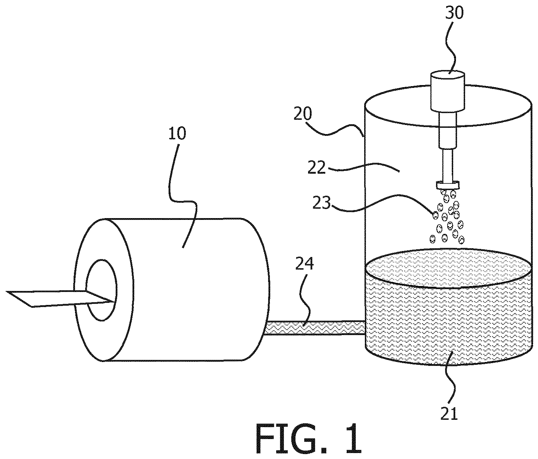

Fig. 1 depicts a commonly known cryo-system for an MRI system. An MRI imager 10 comprises a superconducting magnet (not shown) that is cooled using liquid helium that flows along the magnet. The liquid helium is transported to and from the MRI imager 10 through conduits 24 from a liquid helium reservoir 20 filled with liquid helium 21 at a temperature below the boiling point of helium (< 4.2K). Because the liquid helium returning from the MRI imager has heated up due to its proximity to strongly heat-emitting components the temperature of the liquid helium 21 in the reservoir increases causing helium to evaporate into its gaseous form 22. The gaseous helium 22 escapes from the liquid helium 21 to occupy the volume in the reservoir above the liquid helium 21 surface. When the pressure in the reservoir 20 increases too high the gaseous helium 22 needs to be vented and new, costly liquid helium needs to be added to the reservoir. Also variations in liquid helium temperature and pressure influences the cooling capacity and may cause irregular or even unstable operation of the superconducting magnet.

To prevent, or at least reduce, this undesired effect, a cold-head 30 is installed that protrudes into the reservoir 20 in an area above the liquid helium 21 surface. The cold-head is essentially a rod at a temperature below 4.2K that condensates gaseous helium 22 that comes into contact with it and the re-liquefied helium 23 rains down into the liquid helium 21 volume. As such the amount of liquid helium is kept at a higher level and more stable temperature, thereby cooling of the magnet more efficiently with less fluctuations. Also less new liquid helium needs to be added, which is economically beneficial.

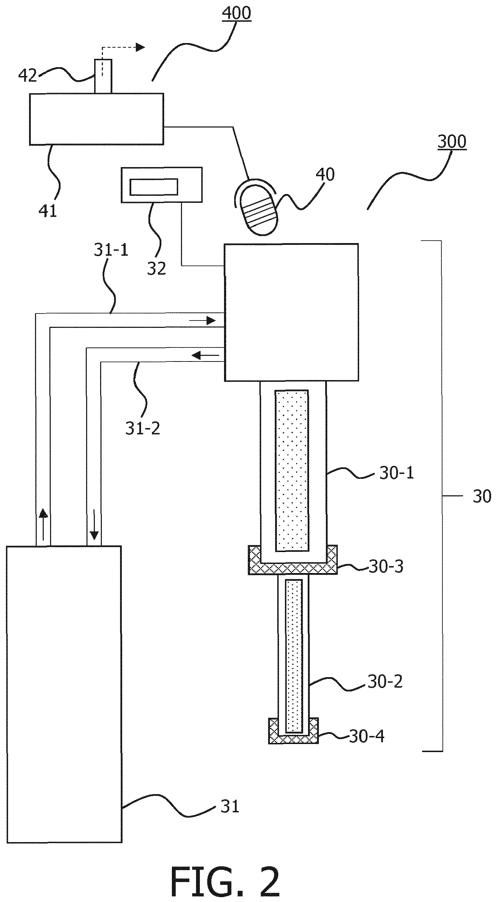

Fig. 2 shows a cryo-system 300 and some main supporting equipment in more detail. The cryo-system 300 itself comprises a cold-head 30 (also known as cold-finger or expander) and a compressor 31. The compressor 31 supplies high pressure helium through a high pressure helium conduit 31-1 to the cold-head 30, where a so-called here Gifford-McMahon refrigeration cycle takes place, which cools the compressed helium to a temperature near the absolute freezing point by controllably expanding the high pressure helium gas using valves, thereby cooling it down. This is usually done in two stages 30-1, 30-2. At least parts of the cold-head is highly shielded (not shown) from temperature or convection to maintain the low temperature. Temperature may be controlled using a temperature control 32. One or more cold tips 30-3, 30-4 are thermally connected to the cooled helium inside the cold-head stages 30-1, 30-2 and are exposed to gaseous helium 22 in the reservoir 20 in which the cold-head 30 is installed. Gaseous helium condenses against the cold tip(s) 30-3, 30-4 and drops back into the liquid helium 21 below. Low-pressure helium returns to the compressor 31 through a low-pressure helium conduit 31-2.

Due to the extreme temperature circumstances the cold-head 30 needs to be regularly checked and/or serviced and in case of inadequate operation it needs to be replaced to prevent loss of cooling capacity to the magnet and of loss of liquid helium, as well as reducing risks associated with a build-up of pressure in the cryo-system.

A servicing engineer, who will check the cold-head 30 regularly or in case of sudden malfunction, will visit the site of the cold-head and will normally service or replace the cold-head 30.

The regular servicing checks will improve operational lifetime of the cold-head, but they are a momentary check and the cold-head may actually be serviced or replaced while operation is still good or the cold-head 30 may malfunction shortly after the servicing engineer's visit, requiring him to return to again service or replace it. On the other hand a cold head may have performed sub-optimally for an unnecessary period until the next visit of the service engineer is scheduled. In any case, such regular checks are virtually never exactly timed at the most optimal moment and may be a waste of time, money, efficiency and operational quality of the MRI scanner.

The presently claimed invention provides a way of continuously monitoring the cold-head 30 and to make sure that a service engineer is only called in when necessary, i.e. when the cold-head 30 first starts showing signs of sub-optimal performance, but well before this will result in significant helium loss or operational degradation.

It is an insight underlying the presently claimed invention that a sub-optimal cold-head performance may be detected because its normal operational sound changes, e.g. unusual noises appear, a frequency of the sound changes, sound gets louder or softer or even stops, etc. a sound detection device 40, i.e. a microphone, with sufficient sensitivity is placed such that it is positioned to detect sound generated by the cold-head 30. Preferably the sound detection device 40 is configured and positioned such that it does not significantly detect sound from or is disturbed by sources other than the cold-head 30. This may be achieved by positioning the sound detection evice 40 as close as possible to the cold-head 30, provide sound shielding for the sound detection device 40 and/or arrange for the sound detection device to be strongly uni-directional aimed at the cold-head 30, e.g. by using a directional microphone. In another embodiment the sound detection device 40 is placed close to a hollow volume that is acoustically connected to the cold-head 30, such as the liquid helium reservoir 20 or the magnet helium chamber (i.e. placing and/or aiming the sound detection device at the side of the magnet). Such hollow volumes amplifies sound and may even be a stronger indicator of sub-optimal cold-head 30. Obviously care must be taken that other operational or ambient sounds must be filtered or avoided to be able to detect sound form the cold-head 30 exclusively.

The sound detection device 40 may operate continuously or intermittently or in another embodiment only when requested locally or remotely by a service engineer or other operator.

The sound detected by the sound detection device 30 is transmitted, by wire or wireless, to a sound analyzer 41, e.g. a computer processor equipped to receive sound information, process the sound information and analyze the sound information. The sound analyzer 41 may be located at or near the site of the cold-head 30 and sound detection device 40. Alternatively it may be located at a remote location, for instance at a data processing center or at a location of a servicing engineer, where one sound analyzer 41 may receive sound information from a plurality of sound detection devices 40 detecting sound form different cold-heads 30 at various locations. The detected sound comprises one or more sound characteristics, such as frequency, loudness, peaks, drop-outs, repeating patterns, etc. The sound analyzer 41 is configured to analyze the detected sound and identify the one or more sound characteristics therein.

The sound analyzer may analyze the detected sound and determine the one or more sound characteristics 41 continuously, but preferably the sound characteristics are determined at pre-determined regular intervals and/or when requested by maintenance or servicing engineer. Continuous determination has the advantage that any potential deviating sound characteristic indicating sub-optimal cold-head 30 performance is directly detected in real time. An advantage of intermittent sound detection and analysis is that computational burden is lowered and/or, for instance, the sound analyzer 41 may alternatingly analyze sounds from multiple cold-heads 30 at various locations.

To determine if the detected sound deviates from normal operation, a calibration or base-line sound recording must be made when the cold-head 30 is known to be operating normally. As such reference one or more sound characteristics are determined that are associated with normal cold-head 30 operation. When the sound analyzer 31 detects a deviation in sound characteristics of the detected sound from the reference sound characteristics then this may indicate sub-optimal operation. Deviations may be a deviation in frequency or volume, a silence or an additional single, continuous or intermittent sound that is not present during normal operation of the cold-head 30.

Preferably the sound analyzer 30 is configured to filter out or disregard non-relevant sounds, such as sounds generated by persons or equipment in the vicinity of the cold-head 30.

Since helium gas acts as a sound insulator and liquid helium is a good sound conductor, the sound originating from the cold-head is conducted and/or absorbed differently when the ratio between gaseous and liquid helium changes, for instance due to sub-optimal cold-head 30 operation. Sound characteristics, e.g. the sound frequency, from the cold-head 30 therefore may gradually change and this may be an indication of predicting when cold-head 30 operation will change beyond a predetermined level and servicing may be pro-actively scheduled. Also, if the rate of sound characteristics change is monitored then an increased rate of change may indicate a more acute problem.

As there may be minor differences in sound characteristics even during normal operation of the cold-head 30 it is preferable to only issue a notification or alarm when the one or more sound characteristic deviates outside a pre-determined range, for instance 10%, 20% or 30% outside of such a range. In a further preferred embodiment the sound analyzer 41 may generate a warning if one or more sound characteristics deviate for an extended period of time or if a pre-determined amount of multiple occurrences, e.g. peak sounds or drop-out noises, occur outside the pre-determined range within a predetermined amount of time.

The sound analyzer 41 may analyze using sound intervals corresponding with detected sound received within a period of time of a predetermined length, for instance a period of time of 5 minutes or of 10 minutes or a time shorter, longer or in between.

In case the sound analyzer 41 is configured to continuously receive detected sound from the sound detection device 40, then the incoming detected sound may be divided into such sound intervals, wherein the sound characteristics of one interval are analyzed and normal or sub-optimal operation of the cold-head 30 is determined within that interval. After the interval has passed a new interval starts and a new sound analysis and state of cold-head 30 operation is determined.

In case the sound analyzer 41 operates intermittently, on regular or irregular intervals or by request, then one or more sound intervals may be analyzed in succession. In an embodiment a first interval may be short, e.g. 1, 2 or 5 minutes. If no sound characteristics are detected that are outside the range of the reference sound characteristics of the cold-head 30 then the analysis may end and, for instance, in case the sound analyzer 41 analyzes sound from multiple cold-heads 30, another cold-head 30 may be checked. If sound characteristics are detected with the first interval that may indicate sub-optimal performance of the cold-head 30, then a second, potentially longer, interval may be analyzed 30. More intervals may also be analyzed if necessary or desired.

The result of the sound analysis by the sound analyzer 31 may be transmitted to a maintenance or service engineer (and/or any other suitable person on site of the cold-head 30 or remote) using a transmitter 42, which may be a wired or wireless transmitter 42 using phone, radio, internet, wifi, bluetooth or any other commonly known manner of transmitting information.

The result may be a report indicating one or more of the following: alert in case sub-optimal cold-head 30 performance is suspected, confirmation of normal cold-head 30 operation in case no deviating sound characteristics were detected, severity of any deviating sound characteristic, a list or graphic depiction of values relating to one or more determined sound characteristics, identification information of the cold-head 30 that was checked, time since the previous check, an overview or a selection of analysis results of past checks, and any other information that may be useful to a service engineer with respect to the checked cold-head 30.

Preferably the transmitter transmits a notable alert when the sound characteristic is outside the pre-determined range and sub-optimal cold-head 30 operation is suspected, for instance a sound, a prominent warning notification, a different color in the report, increased font or any other manner in which an alert would be spotted as soon as possible.

Further, any suitable available information relating to equipment connected to the cold-head 30, such as conduits 24, 31-1, 31-2, helium reservoir 20, compressor 31, temperature control 32, MR imager 10 may be added as well. For instance, in case there was a problem in one of the conduits or other parts of the cryo-system or if the MR imager 10 is requiring a particular high cooling rate, this may have influence on cold-head operation 30, causing it to be required to be operated at more extreme conditions thereby creating sounds that may deviate from normal operation even if the cold-head itself is still in good condition.

Expansion or shrinkage of the liquid helium container, motion of conduits 24, 31-1, 31-2 due to vibrations or other disturbances or technicians, other personal or equipment near the cold-head 30 may also cause sounds to be detected by the sound detection device 40 that may be incorrectly determined as originating from the cold-head 30 and may cause the sound analyzer 41 to falsely determine that the cold-head 30 is not operating within normal parameters. In an embodiment the sound analyzer 41 receives operational information of equipment in the vicinity of the cold-head or information, such as camera images, of personnel present near the cold-head 30. This ambient information may be used as additional input to analyze the sound characteristics from the detected sound and analyze, potentially by using deep learning algorithms, and determine whether any sound characteristic deviating significantly from the reference sound characteristic may be caused by an outside source instead of the cold-head 30 and may disregard these sounds in determining the operational state of the cold-head.

After the notification has been transmitted, it is received by a service or maintenance engineer, for instance through a message on a computer or (mobile) telephone. When the engineer is aware of the operational condition of the cold-head 30, he can decide, amongst others, to schedule a visit to the location of the cold-head 30 to check, service or replace it, to cancel, postpone or pull forward a previously planned visit, do nothing and/or request a new sound measurement. As such the engineer only visits the site of the cold-head 30 when necessary, thereby ensuring the correct action at the most optimal time and potentially reduce travel and servicing or replacement costs. Regularly scheduled servicing checks may still be done, for instance when the maintenance or service engineer regularly checks other equipment on site as well, but he may reschedule these visits based on needs and other required work, particularly if there is no need to service or replace the cold-head.

The workflow of checking and servicing a cold-head may be further optimized by automation. A cold-head servicing workflow organizer, preferably implemented on a computer processor, receives operational information from one or more cold-heads 30 from cold-head monitoring system according to the presently claimed invention. Then it is determined automatically if a visit of a servicing or maintenance engineer is necessary. A report is provided for the engineer and in case servicing is deemed necessary, the service engineer is notified of this. The engineer can then schedule a visit to service, repair or replace the cold-head 30.

The cold-head servicing workflow organizer may receive operational information from a plurality of cold-heads 30 at different locations within one imaging center or between, even widely geographically separated, different imaging centers. The organizer may then determine which cold-heads 30 need to be prioritized in servicing and which may be left unvisited for the time being. The cold-head servicing workflow organizer may then suggest or actually automatically (re-)schedule one or more visits to service one or multiple cold-heads. Prioritization may be based on geographical location of the cold-head(s) and/or cold-heads and urgency of necessary servicing, whereby servicing multiple cold-heads 30 in the same location or close to each other would be preferable.

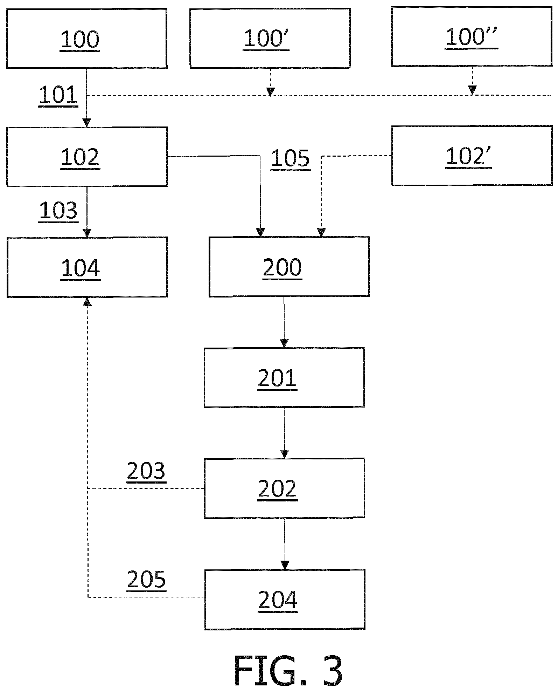

Fig. 3 shows a simple workflow to illustrate a method of monitoring a cold-head 30according to the presently claimed invention. First sound is detected (100) from a cold-head 30, which is transmitted (101), by wire or wirelessly, to a sound analyzer 40, which analyzes (102) one or more sound characteristics of the detected sound from the cold-head 30. Next the analyzed sound characteristic and/or a report thereof is transmitted (103), by wire or wirelessly to a maintenance or servicing engineer. Sound from multiple sound detection devices 30 may be detected (100, 100', 100") and transmitted (101) to the sound analyzer 40, which analyzes (102) each separately and transmits (103) sound characteristics separately or jointly to the service or maintenance engineer. In Fig. 3, sound detection from three cold-heads is shown, but this may be more or less. The service or maintenance engineer receives (104) the one or more sound characteristics and/or the report.

In an embodiment the sound analyzer 40 also transmits (105), by wire or wirelessly, analyzed sound characteristics or operational information of a cold-head to a cold-head servicing workflow organizer, which receives (200) the sound characteristics and/or a report thereof. The workflow organizer determines (201) whether servicing is necessary based on the received operational information, e.g. by determining whether the received sound characteristic is outside a pre-determined range of a predetermined reference sound characteristic of the cold-head 30 that is associated with normal operation. A notification unit of the workflow organizer transmits (203) a report to the service engineer that includes whether the cold-head 30 requires servicing or not. Optionally a report is only transmitted (203) in case servicing is required.

In a further embodiment the workflow organizer also includes a scheduling unit which schedules (204) one or more visits to service the cold-head 30 in case servicing is required and transmits (205) the schedule, which is received (104)by the service or maintenance engineer. It may be included in the report with the analyzed sound characteristics.

Preferably the workflow organizer receives operational information or analyzed sound characteristics relating to a plurality of cold-heads 30 from different cooling systems (102, 102') that may be geographically separated from each other and determines (201) servicing is necessary for one or more cold-heads 30. Only two are shown in Fig. 3, but this may be more or less. Preferably the workflow organizer schedules (204) an optimal visit schedule for one or more visits to service one or multiple cold-heads (30) based on geographical location of the cold-head(s) 30 and urgency of necessary servicing. Preferably cold-heads that need to be serviced that are in proximity of each other (e.g. in the same facility or same area are scheduled (204) to be visited together to reduce travel time and costs. Cold-heads 30 for which it was determined that servicing is urgently required may be scheduled (204) to be visited with priority.

The workflow organizer transmits (203) the sound characteristics and/or a report thereof, which is received (104) by the service or maintenance engineer.

An advantage of the presently claimed monitoring system and method is that it is suitable for any device using a cold-head 30 and is not restricted to just MRI or even medical devices. It is also vendor or manufacturer independent and one sound analyzer 40 may be used to analyze detected sound from cold-heads 30 used in different types of devices or from different manufacturers.

The sound analyzer 40 and the cold-head servicing workflow organizer may be implemented as software or computer program running on a computer or workstation, preferably on the same computer or workstation.

The term service engineer or maintenance engineer or any other similar term known in the field may be used to describe anyone qualified to service a cold-head 30. It may also be a team of engineers. The engineer may be present on-site of the cold-head 30 or maybe at a remote location, for instance at a service providing organization, e.g. one that is part of the manufacturer of the cold-head, the cooling system or the device or system to be cooled.

The terms cooling system, cryogenic and cryo-system and the like are used interchangeably.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A cold-head monitoring system (400) for monitoring operation of a cold-head (30) of a superconducting magnet cooling device (300), comprising:
a sound detection device (4) positioned such that it is able to detect sound generated by the cold-head;
a sound analyzer (41) that is configured to analyze a sound characteristic of the detected sound from the cold-head; and
a transmitter (42) arranged to transmit the determined analyzed sound characteristics and/or a report thereof to a maintenance or servicing engineer.

2. A system according to claim 1, wherein the sound analyzer (41) is configured to analyze the sound characteristics at pre-determined regular intervals and/or when requested by maintenance or servicing engineer.

3. A system according to claim 1 or 2, wherein the sound analyzer (41) is configured to determine if the sound characteristic is within a pre-determined range of a predetermined reference sound characteristic of the cold-head that is associated with normal operation.

4. A system according to claim 3, wherein the transmitter (42) transmits a notable alert when the sound characteristic is outside the pre-determined range.

5. A system according to any of the previous claims, wherein the sound characteristic includes any of the following: a frequency, a volume, a silence, an additional single, continuous or intermittent sound that is not present during normal operation of the cold-head.

6. A system according to any of the previous claims, wherein the sound analyzer (41) is configured to analyze the detected sound during a time interval with a predetermined length, preferably a length less than 10 minutes, more preferably between 5 and 10 minutes.

7. A system according to any of the previous claims, wherein the sound detection device (40) is arranged to detect sound from the cold-head (30) with as little as possible ambient sounds not generated by the cold-head, for instance by using a directional microphone aimed at the sound-head and/or shielding provided to shield the ambient sounds from reaching the sound detection device.

8. A system according to any of the previous claims, wherein the sound analyzer (41) is configured to separate ambient sounds not generated by the cold-head (30) from sound generated by the cold-head and then preferably disregard the ambient sounds in the analysis of the detected sound and/or include information of the ambient sounds in the information to be transmitted by the transmitter (42)

9. A cooling system for cooling a superconducting magnet comprising:
a cooling device (300) comprising a cold-head (30); and
a cold-head monitoring system (400) for monitoring operation of the cold-head (30) according to any of the previous claims.

10. A cooling system according to claim 9, wherein the cooling device (300) further comprises a compressor (31) for compressing helium (31) and to supply pressurized, preferably liquid, helium to the cold-head (30) through a high pressure helium conduit (31-1) and to receive depressurized helium from the cold-head through a low pressure helium conduit (31-2).

11. A magnetic resonance imaging system comprising:
a magnetic resonance imager (10) comprising a superconducting magnet;
a cooling system according to claim 8 or 9.

12. A method for monitoring operation of a cold-head (30) of a superconducting magnet cooling device (300), comprising:
detecting (100, 00', 100") a sound generated by the cold-head;
analyze (102) a sound characteristic of the detected sound from the cold-head; and
transmit (103) the determined analyzed sound characteristics and/or a report thereof to a maintenance or servicing engineer.

13. A cold-head servicing workflow organizer comprising:
a receiver for receiving (200) operational information from a cold-head of a cooling system for cooling for a superconducting magnet, wherein the operational information is supplied by a cold-head monitoring system according to any of the claims 1 to 6;
a determination unit for determining (201) based on the received operational information whether servicing is necessary; and
a notification unit for providing (202) a report about the operational information and in case servicing was deemed necessary, notifying a service engineer that servicing of the cold-head is required.

14. A cold-head servicing workflow organizer according to claim 12, wherein the receiver is arranged to receive (200) operational information from at least two cold-heads of different cooling systems.

15. A cold-head servicing workflow organizer according to claim 13, further comprising:
a scheduling unit arranged to schedule (204) one or more visits to service one or multiple cold-heads based on geographical location of the cold-head or cold-heads and urgency of necessary servicing.
